# EUROPEAN PATENT SPECIFICATION

(11) **EP 4 036 245 B1**
(45) Date of publication and mention of the grant of the patent: **11.06.2025**
(21) Application number: 20867839.1
(22) Date of filing: 17.09.2020
(51) Int. Cl.: B01D 15/32, C12P 19/04, B01J 20/287, C12N 1/20, C08B 37/00, C08L 5/00

(54) **LIPOPOLYSACCHARIDE PRODUCTION METHOD**
VERFAHREN ZUR HERSTELLUNG VON LIPOPOLYSACCHARID
PROCÉDÉ DE PRODUCTION DE LIPOPOLYSACCHARIDES

(30) Priority: 24.09.2019 JP 2019173207
(43) Date of publication of application: 03.08.2022
(73) Proprietor: Biomedical Research Group Inc., 158-0084 Tokyo (JP); Macrophi Inc., Takamatsu-shi, Kagawa 7610301 (JP)
(72) Inventor: SOMA, Gen-Ichiro, Tokyo 158-0084 (JP); INAGAWA, Hiroyuki, Takamatsu-shi, Kagawa 761-0301 (JP); KOHCHI, Chie, Takamatsu-shi, Kagawa 761-0301 (JP); MATSUSHITA, Kyohei, Ibaraki-shi, Osaka 567-8680 (JP); HASHINO, Ryo, Ibaraki-shi, Osaka 567-8680 (JP); LI, Wenjing, Ibaraki-shi, Osaka 567-8680 (JP)
(74) Representative: Appelt, Christian W.
(86) International application number: PCT/JP2020/035204
(87) International publication number: WO 2021/060127

(56) References cited:
- EP-A1- 1 961 823
- WO-A1-2007/061102
- WO-A1-96/23002
- WO-A1-96/23002
- JP-A- 2010 145 191
- JP-A- 2010 145 191
- JP-A- 2018 035 084
- KOL O ET AL: "Purification of the lipopolysaccharide fraction from Klebsiella pneumoniae O"1 K"2 by high-performance liquid chromatography", JOURNAL OF CHROMATOGRAPHY A, ELSEVIER, AMSTERDAM, NL, vol. 396, 1 January 1987 (1987-01-01), pages 281 - 286, XP026554771, ISSN: 0021-9673, [retrieved on 19870101], DOI: 10.1016/S0021-9673(01)94065-8
- CHRISTIAN R H RAETZ ET AL: "Kdo2-Lipid A of Escherichia coli, a defined endotoxin that activates macrophages via TLR-4", JOURNAL OF LIPID RESEARCH, AMERICAN SOCIETY FOR BIOCHEMISTRY AND MOLECULAR BIOLOGY, INC, US, vol. 47, no. 5, 1 May 2006 (2006-05-01), pages 1097 - 1111, XP002651695, ISSN: 0022-2275, [retrieved on 20060214], DOI: 10.1194/JLR.M600027-JLR200
- NISHIZAWA, TAKASHI ET AL.: "Examination on the biological activity of lipopolysaccharides of a lowmolecular-weight fraction and high-molecularweight fraction of lipopolysaccharides derived from Pantoea Agglomerans", BIOTHERAPY, vol. 10, no. 3, 30 November 1995 (1995-11-30), JP , pages 519 - 521, XP009535332, ISSN: 0914-2223
- RAETZ C. R. H. ET AL.: "Kdo2-lipid A of Escherichia coli, a defined endotoxin that activates macrophages via TLR-4", JOURNAL OF LIPID RESEARCH, vol. 47, 2006, pages 1097 - 1111, XP002651695, DOI: 10.1194/jlr.M600027-JLR200

## Description

### [Technical Field]

The present invention relates to a lipopolysaccharide production method.

### [Background Art]

A lipopolysaccharide (may also be referred to hereinafter as "LPS") is a composite compound constituted of a lipid and a saccharide and is present in the outer membrane that surrounds the peptidoglycan of cell walls of gram-negative bacteria such as *Escherichia coli, Salmonella enterica, Bordetella pertussis,* etc., and is known as an active component of endotoxins (NPL 1).

The basic structure of the abovementioned LPS is constituted of the three components of lipid A that has a specific lipid, an oligosaccharide called R core that is covalently bonded thereto, and further, an O-specific polysaccharide (NPL 2). Also, it is known that the structure of an LPS differs according to the type of bacteria from which it is derived.

Although LPS are known as endotoxins and known to activate immune cells and increase inflammatory cytokines, the function of LPS that activates immune cells has been attracting attention recently. Innate immunity research and biological defense mechanism research using LPS have thus come to be performed actively. Meanwhile, LPS derived from *Pantoea* bacteria has a rich history of use in human foods, has also been reported for its possibility of use in health foods, vaccine adjuvants, pharmaceuticals, etc., and can thus be said to be an especially useful LPS.

Although, demand for LPS is thus increasing, in regard to production of LPS, since large amounts of organic solvents such as trichloroacetic acid, phenol, chloroform, toluene, alkane, petroleum ether, etc., are used as extraction solvents in its extraction step, the health of production workers is harmed and impact on the natural environment due to discharge is also presenting a problem. Also, it is necessary to introduce special equipment compatible with the abovementioned organic solvents and the burden of production cost, etc., is thus a problem.

For example, as a known LPS production method, the Galanos method is known and this is a method that includes LPS extraction by a mixture of phenol, chloroform, and petroleum ether (PCP) followed by evaporation of chloroform and petroleum ether, precipitation of LPS by addition of acetone and water, and recovery of LPS by centrifugation or filtering (NPL 3).

Also, in the Tsang method, an LPS is obtained by performing extraction by a trichloroacetic acid aqueous solution and thereafter performing extraction by a phenol aqueous solution (NPL 4).

Also, the Chen method includes LPS extraction by a mixture of chloroform and methanol (CM) followed by a series of methanol precipitation steps (NPL 5).

The abovementioned Galanos method and Tsang method use solvent mixtures (phenol, chloroform, petroleum ether, trichloroacetic acid, etc.) with which there are environmental, health, and safety concerns in the extraction step and are thus unfit for large-scale production and unsuitable for commercial production. Also, even with the Chen method, chloroform is used in the extraction step and there are thus environmental, health, and safety concerns, and also with the Chen method, since a CM phase that is rich in LPS-phospholipid is formed, multiple precipitation steps are required to obtain an LPS of sufficient purity and production time and cost are thus required.

Also, for example, it is disclosed in PTL 1 that in a step of extracting an LPS from gram-negative bacteria, an alcohol, an organic solvent, and water are used in place of the solvent mixture of phenol, chloroform, and petroleum ether (PCP) used in the Galanos method.

However, it is disclosed that as the abovementioned organic solvent, an organic solvent that can be selected from the group consisting of chloroform, alkanes, toluene, and petroleum ether is used and thus even this production method has environmental, health, and safety concerns and is unfit for large-scale production.

The conventional LPS production methods thus include production processes that are high in burdens on the environment and labor hygiene. In other words, the conventional LPS production methods have risks of environmental conservation in addition to risks of labor hygiene management i.e., working environment management, work management, and health management. Also, LPS production facilities are deemed to require countermeasure equipment for the abovementioned risks and production cost is thus high.

Also, among LPS, *Pantoea* (may also be referred to hereinafter as *"Pantoea* bacteria") derived LPS, which is useful in health food, vaccine adjuvant, pharmaceutical applications, have been reported variously for its benefits as mentioned above and increase in demand is anticipated. Development of a production method that does not use an organic solvent in an extraction step and is suitable for large-scale production is thus being demanded in particular. However, in regard to a *Pantoea* bacteria derived LPS production method, there is just a report on a method that combines extraction by hot phenol and anion exchange column purification (NPL 6), and since this method also uses phenol, there are health and safety concerns in performing large-scale production.

An LPS production method suited for large-scale production, in other words, a production method that is low in burdens of labor hygiene and environmental conservation is thus demanded. Such a production method is demanded especially in regard to production of *Pantoea* bacteria derived LPS.

Here, as an example of an LPS extraction method that does not use a harmful organic solvent, it is disclosed in PTL 2 to perform hot water extraction on *Acetobacter* bacteria, *Gluconobacter* bacteria, *Xanthomonas* bacteria, *Zymomonas* bacteria, or *Enterobacter* bacteria. However, the lipopolysaccharide (LPS) obtained by this production method contains large amounts of impurities other than the LPS and the method is merely an extraction operation for obtaining an extract of low LPS purity and cannot be regarded as a practical method for producing LPS. Also, although an LPS production method is separately disclosed in PTL 2, that method combines hot phenol extraction and nuclease treatment. In other words, a method for producing LPS without using a harmful organic solvent is neither disclosed nor suggested in PTL 2.

Also, ordinarily, a purified extract of LPS is a mixture of a low molecular weight component and a high molecular weight component and it has been reported that when the ratio of the low molecular weight LPS increases, safety increases and physiological activity (cytokine inducibility) also improves (PTL 3, NPL 7). An LPS that contains more low molecular weight LPS than an LPS produced by conventional production methods and a method for producing such an LPS are thus demanded.

### [Citation List]

### [Patent Literature]

[PTL 1] JP 2016-174601 A
[PTL 2] WO 2007/061102 A1
[PTL 3] JP 4043533 B2

### [Non Patent Literature]

[NPL1] Edited by J. M. Ghuysen and R. Hakenbeck, Bacterial Cell Wall, New Comprehensive Biochemistry, Elsevea, 1994, Vol. 27., p.18.
[NPL 2] Nikkei Baiotekunolojii Saishin Yogo Jiten, Nikkei McGraw-Hill Inc., 1985, p.431.
[NPL 3] Galanos et al., Eur. J. Biochem., 1969, 9, p.245.
[NPL 4] J. C. TSANG et al., JOURNAL OF BACTERIOLOGY, Feb. 1974, p.786-795.
[NPL 5] CHEN et al., Journal of Infectious Diseases, 1973, 128s, p.43.
[NPL 6] ANTICANCER RESEARCH, 2007, 27, p.3701-3706.
[NPL 7] Biotherapy, March 1996, 10(3), p.519-521.

### [Summary of Invention]

### [Technical Problem]

In view of the current circumstances described above, an object of the present invention is to provide a lipopolysaccharide production method that contributes to labor hygiene and environmental conservation and is suited for large-scale production and a lipopolysaccharide produced thereby.

### [Solution to Problem]

To achieve the above object, intensive study was performed on a method for extracting and purifying an LPS by which a yield enabling application to large-scale production can be obtained without using an organic solvent of high environmental burden. As a result, it was found that by combining a first step of performing extraction from gram-negative bacterium using hot water with a temperature of 50 to 150°C and a second step of purifying an extract liquid obtained in the first step or a solution containing an LPS in the extract liquid by using reverse-phase liquid chromatography provided with a reverse-phase column having a specific packing material, an LPS of a yield enabling application to large-scale production can be obtained without using an organic solvent of high environmental burden. Further, it was found that the LPS obtained by the present production method is high in purity and contains a high content of low molecular weight lipopolysaccharide.

That is, the present invention is a lipopolysaccharide production method in which a lipopolysaccharide is extracted and purified from gram-negative bacterium and is a lipopolysaccharide production method that is characterized in including a first step of performing extraction from the gram-negative bacterium using hot water to obtain an extract liquid that contains the lipopolysaccharide, wherein a temperature of the hot water is 50 to 150°C, and a second step of purifying the extract liquid or a solution containing the LPS in the extract liquid by using reverse-phase liquid chromatography to obtain the lipopolysaccharide and in that a reverse-phase column used in the reverse-phase liquid chromatography has a packing material constituted of a material having a functional group of 1 to 8 carbons.

The packing material is preferably constituted of a material having a functional group of 2 to 6 carbons.

The packing material is preferably constituted of a material having a functional group of 2 to 4 carbons.

The packing material is preferably constituted of a material having a functional group of 4 carbons.

Also, the functional group is preferably an alkyl group.

A temperature of the hot water of the first step is 50 to 150°C.

The temperature of the hot water of the first step is preferably 50 to 99°C.

The temperature of the hot water of the first step is preferably 70 to 99°C.

The temperature of the hot water of the first step is preferably 85 to 95°C.

The gram-negative bacterium is preferably at least one selected from the group consisting of a genus *Escherichia, Salmonella, Pantoea, Acetobacter, Zymomonas, Xanthomonas, Enterobacter, Roseomonas,* and *Rhodobactor.*

The gram-negative bacterium preferably belongs to the genus *Pantoea.*

Also disclosed, however not forming part of the present invention, is a lipopolysaccharide characterized in being produced by the production method of the present invention.

Also disclosed, however not forming part of the present invention, is a lipopolysaccharide preferably obtained from the abovementioned gram-negative bacterium.

Also disclosed, however not forming part of the present invention, is a lipopolysaccharide that contains a low molecular weight lipopolysaccharide with a molecular weight as measured by an SDS-PAGE method of 2,000 to 20,000 and a high molecular weight lipopolysaccharide with a molecular weight as measured by the SDS-PAGE method of more than 20,000 but not more than 100,000 and a content of the low molecular weight lipopolysaccharide with respect to a total amount of the low molecular weight lipopolysaccharide and the high molecular weight lipopolysaccharide is not less than 80%.

Also disclosed, however not forming part of the present invention, is a lipopolysaccharide that is preferably such that a value (E/L ratio) obtained by dividing a lipopolysaccharide quantitative value (E) determined by an ELISA method by a lipopolysaccharide quantitative value (L) determined by a Limulus test (endpoint chromogenic method) is not more than 1.0.

The present invention shall now be described in detail. Here, in the following description, a percentage indication indicates a value by weight unless noted otherwise.

### [Advantageous Effects of Invention]

By the lipopolysaccharide production method of the present invention, a lipopolysaccharide production method that contributes to labor hygiene and environmental conservation and is suited for large-scale production can be provided.

To contribute to labor hygiene and environmental conservation specifically means that a lipopolysaccharide can be extracted without using an organic solvent of high environmental burden, and environmental, health, and safety risks can thereby be improved. Also, since in the production of the lipopolysaccharide, risks of environmental conservation as well as risks of labor hygiene management i.e., working environment management, work management, and health management can be reduced, countermeasure equipment for the abovementioned risks in LPS production facilities can be reduced and overall production cost can be suppressed.

Further, by the production method of the present invention, a lipopolysaccharide of high purity and high content of low molecular weight lipopolysaccharide is obtained, thus making it possible to provide a lipopolysaccharide that is high in safety and physiological activity.

### [Brief Description of Drawings]

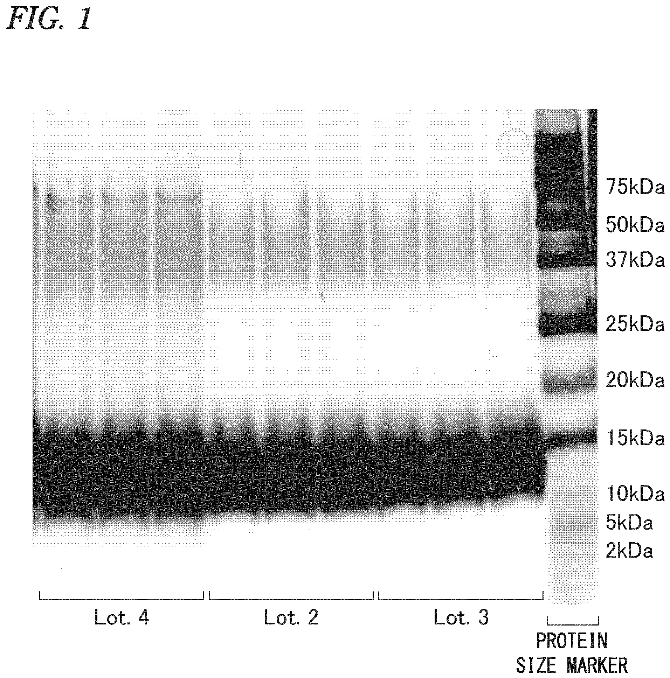

[FIG. 1] FIG. 1 is an SDS-PAGE (silver staining method) electrophoretogram.

### [Description of Embodiments]

In the present Description, the abovementioned solvent of high environmental burden is, in regard to a first step of obtaining an extract liquid that contains an LPS, an organic solvent and further, in regard to an entirety of a manufacturing process excluding the first step, is a substance that is designated as 'Class I designated chemical substance' in 'Act on Confirmation, etc. of Release Amounts of Specific Chemical Substances in the Environment and Promotion of Improvements to the Management Thereof' and is usable as an organic solvent.

In the present Description, an LPS of high purity means an LPS in which an LPS is a main component in comparison to proteins and nucleic acids, that is, an LPS with which an LPS content (proportion of the remainder after subtraction of contents (weights) of proteins and nucleic acids from a weight of the LPS obtained) is not less than 60 weight %. Also, in the present Description, "purity" means the purity of an LPS and is expressed in weight %. The purity of an LPS is preferably not less than 70 weight %, more preferably not less than 80 weight %, and even more preferably not less than 90 weight %.

Also, in the present Description, a low molecular weight lipopolysaccharide (may also be referred to hereinafter as "low molecular weight LPS") is an LPS with a molecular weight of not less than 2,000 and not more than 20,000 as determined by development into constituents by SDS-PAGE (silver staining method) and using a protein size marker mobility as an index, and a high molecular weight lipopolysaccharide (may also be referred to hereinafter as "high molecular weight LPS") is an LPS with a molecular weight of more than 20,000 but not more than 100,000 as determined by the abovementioned measurement.

Also, an LPS that contains a high content of a low molecular weight lipopolysaccharide (low molecular weight LPS) means that which practically does not contain a high molecular weight lipopolysaccharide (high molecular weight LPS) as well as that with which, even when a high molecular weight LPS is contained, the content of the low molecular weight LPS with respect to a total of the high molecular weight LPS and the low molecular weight LPS is not less than 80%. An LPS with which the content of the low molecular weight LPS with respect to the total of the high molecular weight LPS and the low molecular weight LPS is not less than 80% is higher in the content of the low molecular weight LPS in comparison to an LPS purified by a conventional method. To describe more specifically, the above means that when molecular weights are determined by development of an LPS into constituents by SDS-PAGE (silver staining method) and use of a protein size marker mobility as an index and contents of the constituents are determined by total image luminance values of an SDS-PAGE image (after silver staining), a content, in the LPS that contains a low molecular weight lipopolysaccharide with a molecular weight of 2,000 to 20,000 and a high molecular weight lipopolysaccharide with a molecular weight more than 20,000 but not more than 100,000, of the low molecular weight lipopolysaccharide with respect to a total of an amount of the low molecular weight lipopolysaccharide and an amount of the high molecular weight lipopolysaccharide as determined by the SDS-PAGE silver staining method is not less than 80%.

In the description that follows, an LPS with which a content of a low molecular weight LPS is not less than 80% shall be expressed as an LPS with a high content of low molecular weight LPS.

Also, in the present Description, a lipopolysaccharide of a yield enabling application to large-scale production refers to a lipopolysaccharide with which a yield of the lipopolysaccharide produced from 1 kg of bacterial cell pellet is not less than 4 g. The yield of the lipopolysaccharide produced from 1 kg of bacterial cell pellet can be measured using an HPLC method.

In the present Description, "A to B" means "not less than A and not more than B."

A lipopolysaccharide production method that is an embodiment of the present invention is a lipopolysaccharide production method in which a lipopolysaccharide is extracted and purified from a gram-negative bacterium and is a lipopolysaccharide production method that is characterized in including a first step of performing extraction from the gram-negative bacterium using hot water with a temperature of 50 to 150°C to obtain an extract liquid that contains the lipopolysaccharide and a second step of purifying the extract liquid or a solution containing the LPS in the extract liquid by using reverse-phase liquid chromatography to obtain the lipopolysaccharide and in that a reverse-phase column used in the reverse-phase liquid chromatography has a packing material constituted of a material having a functional group of 1 to 8 carbons.

In general, an LPS may be an extract from gram-negative bacterial cell walls or a modification product thereof or may be a synthetic product. However, the LPS obtained by the LPS production method of the present invention is a purified extract from gram-negative bacterial cell walls.

As the gram-negative bacteria, for example, those belonging to a genus *Escherichia, Shigella, Salmonella, Yersinia, Viblio, Haemophilus, Pseudomonas, Legionella, Bordetella, Brucella, Francisella, Bacteroides, Neisseria, Chlamydia, Plesiomonas, Prophyromonas, Pantoea, Agrobacterium, Stenortophomonas, Serratia, Leclercia, Rahnella, Acidicaldus, Acidiphilium, Acidisphaera, Acidocella, Acidomonas, Asaia, Belnapia, Craurococcus, Gluconacetobacter, Gluconobacter, Kozakia, Leahibacter, Muricoccus, Neoasaia, Oleomonas, Paracraurococcus, Rhodopila, Roseococcus, Rubritepida, Saccharibacter, Stella, Swaminathania, Teichococcus, Zavarzinia, Achromobacter, Flavobacterium, Enterobacter, Acetobacter, Xanthomonas, Zymomonas, Roseomonas, Rhodobactor, Proteus, Klebsiella, Citrobacter, Acinetobacter,* etc., can be cited.

Among the above, the gram-negative bacterium is preferably at least one selected from the group consisting of the genus *Escherichia, Salmonella, Pantoea (Pantoea* bacteria), *Acetobacter, Zymomonas, Xanthomonas, Enterobacter, Roseomonas,* and *Rhodobactor.* This is because these have been contained in many foods and Kampo medicines since ancient times and safety to a living body is ensured. In particular, *Pantoea* is currently used as health food and an LPS extracted and purified from the genus *Pantoea* can be regarded to be higher in safety and effectiveness in its use. The gram-negative bacterium preferably belongs to the genus *Pantoea.* The production method of the present invention can provide, in production of *Pantoea* (*Pantoea* bacteria) derived LPS for which increase in demand is anticipated, a production method that contributes to labor hygiene and environmental conservation and *Pantoea* bacteria derived LPS of high purity and high content of low molecular weight LPS.

In the first step, by extracting the gram-negative bacterium using hot water with a temperature of 50 to 150°C, the cell walls of the gram-negative bacterium are modified and removed, the LPS in the cell walls and the proteins and nucleic acids inside the cells of the gram-negative bacterium are extracted, and the extract liquid that contains the LPS is obtained.

A temperature of the hot water in the first step is 50 to 150°C. This is because if the temperature of the hot water is higher than 150°C, there is a possibility of occurrence of thermal denaturation of the extracted LPS due to the high temperature and if the temperature is less than 50°C, there is a possibility that modification of the cell walls of the gram-negative bacterium will not occur and the LPS will not be extracted sufficiently.

Also, the temperature of the hot water is more preferably 50 to 130°C, even more preferably 50 to 99°C, yet even more preferably 70 to 99°C, and most preferably 85 to 95°C. This is because the LPS can be extracted with high efficiency. It is also because extraction under ordinary pressure is also possible.

A duration of performing the extraction using the hot water is preferably 10 to 120 minutes. This is because if the extraction duration exceeds 120 minutes, thermal decomposition, etc., of the LPS may occur and if the duration is less than 10 minutes, the LPS may not be extracted sufficiently.

In regard to the temperature and duration of the hot water extraction in the first step, optimal conditions can be selected as suited from the abovementioned hot water temperatures and extraction durations in accordance with the type of gram-negative bacterium used.

Although even when the gram-negative bacterium belongs to the genus *Pantoea,* the hot water temperature and extraction duration in the LPS extraction can be selected from within the ranges mentioned above, the hot water extraction is performed preferably with the conditions of 70 to 99°C and 10 to 120 minutes and more preferably with the conditions of 85 to 95°C and 20 to 30 minutes.

Also, the hot water extraction in the first step may be performed just once or may be performed a plurality of times at the same temperature or at different temperatures. Also, the water used in the hot water extraction is not restricted in particular and water that is ordinarily used in the present field can be used.

Also, the hot water extraction can be performed using a method and a device that is generally used in the present technical field and can be performed under ordinary pressure, reduced pressure, or pressurization.

Also, a surfactant, a chelating agent, an organic acid salt, an inorganic salt, etc., may be added to the water used in the hot water extraction.

In the LPS production method of the embodiment of the present invention, the first step and the second step may be performed successively or another step may be included between the first step and the second step.

When the first step and the second step are performed successively, the extract liquid obtained in the first step is purified in the second step. Also, when another step is included between the first step and the second step, a solution that contains the LPS in the extract liquid obtained in the first step is purified in the second step.

As the other step performed between the first step and the second step, for example, ultrafiltration, enzymatic treatment, ethanol precipitation, dialysis, solid phase extraction, etc., can be cited, and among these, it is preferable to perform ultrafiltration on the extract liquid obtained in the first step. This is because by performing ultrafiltration, the LPS in the extract liquid can be concentrated and, at the same time, low molecular weight impurities (those that pass through the 200,000 Dalton cut-off of an ultrafiltration membrane) can be removed to thereby enable the purification using the reverse-phase liquid chromatography in the second step to be performed with high efficiency. The extract liquid obtained in the first step is preferably ultrafiltered once or a plurality of times. Here, the LPS forms a micelle and becomes large in apparent molecular weight and therefore, its molecular weight does not directly correspond to the molecular weight of the cut-off value of the ultrafiltration membrane.

If in the production method of the present invention, ultrafiltration is performed between the first step and the second step, the ultrafiltration membrane used in the ultrafiltration is that with a molecular weight cutoff of 5,000 to 200,000 Daltons and preferably 8,000 to 100,000 Daltons. More preferably, the molecular weight cutoff is 10,000 to 50,000 Daltons.

Here, the molecular weight cutoff is a term expressing a membrane characteristic that is defined in JIS K 3802 (Technical terms for membranes and membrane processes).

The reverse-phase column used in the reverse-phase liquid chromatography used in the second step has the packing material that is constituted of the material having the functional group of 1 to 8 carbons. This is because, by using the reverse-phase column having the packing material that is constituted of the material having the functional group of the specific number of carbons, impurities other than the LPS that remain in the extract liquid obtained in the first step or in the solution containing the LPS in the extract liquid can be removed to enable purification to an LPS of higher purity. It is also because purification to an LPS of high content of low molecular weight LPS is enabled.

The packing material is preferably constituted of a material having a functional group of 2 to 6 carbons, is more preferably constituted of a material having a functional group of 2 to 4 carbons, and is even more preferably constituted of a material having a functional group of 4 carbons.

The functional group is preferably an alkyl group. This is because the impurities other than the LPS that remain in the extract liquid obtained in the first step can be removed to enable purification to an LPS of higher purity. It is also because the LPS can be purified sufficiently while suppressing the cost of equipment used in the production method of the present invention. The packing material is preferably constituted of a material having an alkyl group of 1 to 8 carbons, is more preferably constituted of a material having an alkyl group of 2 to 6 carbons, is even more preferably constituted of a material having an alkyl group of 2 to 4 carbons, and is most preferably constituted of a material having an alkyl group of 4 carbons. That is, the packing material is most preferably constituted of a material having a butyl group.

The material (base material) that constitutes the packing material is preferably a silica gel. This is because the impurities other than the LPS that remain in the extract liquid obtained in the first step can be removed to enable purification to an LPS of higher purity. It is also because the LPS can be purified sufficiently while suppressing the cost of equipment used in the production method of the present invention. The packing material is preferably a silica gel having a functional group of 1 to 8 carbons, is more preferably a silica gel having a functional group of 2 to 6 carbons, is even more preferably a silica gel having a functional group of 2 to 4 carbons, and is most preferably a silica gel having a functional group of 4 carbons.

The material (base material) that constitutes the packing material is preferably a silica gel that has an alkyl group. This is because the impurities other than the LPS that remain in the extract liquid obtained in the first step can be removed to enable purification to an LPS of higher purity. It is also because the LPS can be purified sufficiently while suppressing the cost of equipment used in the production method of the present invention. The packing material is preferably a silica gel having an alkyl group of 1 to 8 carbons, is more preferably a silica gel having an alkyl group of 2 to 6 carbons, is even more preferably a silica gel having an alkyl group of 2 to 4 carbons, and is most preferably a silica gel having an alkyl group of 4 carbons. That is, the packing material is most preferably a silica gel having a butyl group.

The packing material preferably such that the functional group is an alkyl group and the material is a silica gel and an arrangement that suitably combines the abovementioned arrangements can be adopted.

Also, the second step may include a pretreatment performed on the extract liquid obtained in the first step or the solution containing the LPS in the extract liquid that is to be treated by the reverse-phase liquid chromatography. As the pretreatment, an operation that is ordinarily used in the present field and is suitable for separation by the reverse-phase liquid chromatography can be performed. Specifically, the extract liquid obtained in the first step or the solution containing the LPS in the extract liquid may have a surfactant added thereto to perform a solubilization treatment on the LPS that may then be purified by the reverse-phase liquid chromatography. This is because the separation by the reverse-phase liquid chromatography can be made efficient. The second step preferably includes the abovementioned solubilization treatment as the pretreatment for the reverse-phase liquid chromatography.

The surfactant is preferably an ionic surfactant. This is because the effect of solubilizing the LPS is high. The ionic surfactant is not restricted in particular and although alkylammonium salts, bile acids, fusidic acid, amino acids, fatty acid conjugates of oligopeptides or polypeptides, glyceride esters of amino acids and oligopeptides, glyceride esters of polypeptides, acyl lactylates, monoacetylated tartaric acid esters of monoglycerides, diacetylated tartaric acid esters of monoglycerides, monoacetylated tartaric acid esters of diglycerides, diacetylated tartaric acid esters of diglycerides, succinylated monoglycerides, citric acid esters of monoglycerides, citric acid esters of diglycerides, alginic acid, propylene glycol alginic acid ester, lecithin, hydrogenated lecithin, lysolecithin, hydrogenated lysolecithin, lysophospholipids, phospholipids, alkyl sulfate salts, fatty acids, and pharmacologically acceptable salts of the above, etc., can be cited as examples, it is preferably a bile acid and/or a pharmacologically acceptable salt thereof. This is because the effect of solubilizing the LPS is especially high.

Also, as bile acids and pharmacologically acceptable salts thereof, for example, chenodeoxycholic acid (CDCA), ursodeoxychenodeoxycholic acid (UCDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate, glycodihydrofusidic acid, and pharmacologically acceptable salts of the above (for example, sodium salts), etc., can be cited.

Among the above, the surfactant is preferably deoxycholic acid and/or a pharmacologically acceptable salt thereof. Also, the surfactant is preferably deoxycholic acid and/or a salt thereof. This is because the effect of solubilizing the LPS is especially high.

Also, in the second step in the LPS production method of the present invention, besides the above-described solubilization treatment, various pretreatments that are generally performed on a sample liquid for reverse-phase liquid chromatography such as concentration adjustment, pH adjustment, ionic strength adjustment, organic solvent addition, filtration, dialysis, etc., may be performed as suited on the extract liquid obtained in the first step or the solution containing the LPS in the extract liquid.

Also, the second step may include a posttreatment performed on a separated liquid that is obtained using the reverse-phase liquid chromatography and contains the LPS. As the post-treatment, an operation suited to the separated liquid of the reverse-phase liquid chromatography can be performed and as the operation suited to the separated liquid, separated liquid treatments that are ordinarily used in the technical field to which the present invention belongs can be combined as suited. Specifically, for example, methods making use of differences in solubility such as distillation, salting-out, solvent precipitation methods, etc.; methods making use of differences in molecular weight such as dialysis, ultrafiltration, gel filtration, PAGE, SDS-PAGE, etc.; methods making use of differences in isoelectric point such as isoelectric focusing, etc., and the like can be cited. Among these, it is preferable to perform distillation and/or ultrafiltration and more preferable to perform distillation and ultrafiltration as the posttreatment. This is because impurities contained in the mobile phase of the reverse-phase liquid chromatography in the second step can be removed.

Also, the LPS production method of the embodiment of the present invention may have a step of concentrating or a step of washing the LPS purified in the second step. As the concentrating step and the washing step, treatments that are ordinarily used in the technical field to which the present invention belongs can be combined as suited.

Also, the LPS production method of the embodiment of the present invention may have a step (drying step) of drying the LPS purified in the second step. In many cases, a volatile organic solvent contained in the mobile phase of the reverse-phase liquid chromatography in the second step and water or other solvent are contained in the LPS purified in the second step. The drying step thus desirably includes a step (organic solvent removing step) of removing the volatile organic solvent contained in the mobile phase of the reverse-phase liquid chromatography in the second step by a dry distillation method (evaporation) and/or a step (solvent removing step) of completely removing the water or other solvent by freeze drying. By having such a drying step, handleability of the LPS can be improved.

Also, the above-described LPS production method of the present invention can be used favorably in large-scale production. The yield of the LPS obtained from the LPS production method of the present invention is preferably such that not less than 4 g of lipopolysaccharide are produced from 1 kg of bacterial cell pellet of gram-negative bacterium. The LPS obtained from LPS production method of the present invention can be quantified by an HPLC method.

Also disclosed, however not forming part of the present invention, is a lipopolysaccharide produced by the above-described production method of the present invention. The lipopolysaccharide produced by the production method of the present invention is a high purity LPS with a purity of not less than 60 weight % and preferably the purity is not less than 70 weight %, more preferably the purity is not less than 80 weight %, and even more preferably the purity is not less than 90 weight %.

The lipopolysaccharide that is also disclosed herein is preferably produced from the abovementioned gram-negative bacterium. The gram-negative bacterium is more preferably at least one selected from the group consisting of the genus *Escherichia, Salmonella, Pantoea, Acetobacter, Zymomonas, Xanthomonas, Enterobacter, Roseomonas,* and *Rhodobactor* and more preferably belongs to the genus *Pantoea.* This is because these have been contained in many foods and Kampo medicines since ancient times and safety to a living body is ensured. In particular, *Pantoea* is currently used as health food and an LPS extracted and purified from the genus *Pantoea* can be regarded to be higher in safety and effectiveness in its use.

Preferably, the lipopolysaccharide that is also disclosed herein contains a low molecular weight lipopolysaccharide with a molecular weight as measured by an SDS-PAGE method of 2,000 to 20,000 and a high molecular weight lipopolysaccharide with a molecular weight as measured by the SDS-PAGE method of more than 20,000 but not more than 100,000 and a content of the low molecular weight lipopolysaccharide with respect to a total amount of the low molecular weight lipopolysaccharide and the high molecular weight lipopolysaccharide is not less than 80%. An LPS with a low molecular weight LPS content of not less than 80% is especially useful in that it is extremely high in safety and excellent in bioactivity.

In the present invention, the molecular weights of the LPS are values of molecular weight determined by development into constituents by SDS-PAGE (silver staining method) and use of a protein size marker mobility as an index. The content ratio of the low molecular weight LPS and the high molecular weight LPS is a value obtained by determining the contents of the constituents by total image luminance values of an SDS-PAGE image (after silver staining).

The lipopolysaccharide that is also disclosed, however does not form part of the present invention is preferably such that a value (E/L ratio) obtained by dividing a lipopolysaccharide quantitative value (E) determined by an ELISA method by a lipopolysaccharide quantitative value (L) determined by a Limulus test (endpoint chromogenic method) is not more than 1.0. The E/L ratio is a value greater than 0.

The lipopolysaccharide quantitative value (E) determined by the ELISA method and the lipopolysaccharide quantitative value (L) determined by the Limulus test (endpoint chromogenic method) are measured, for example, by the following methods.

### (Method for quantification of LPS by ELISA method)

The quantity of an LPS by the ELISA method is determined by measuring an LPS content (E) (µg/mg) in a sample by, for example, the ELISA method using a carbohydrate specific antibody for the LPS to be measured (for example, IP-PA1 carbohydrate specific antibody, manufactured by Macrophi Inc.). Specifically, the ELISA measurement can be performed by the following procedure.
1) 34-G2 (immobilized antibody: antibody against O antigen polysaccharide of IP-PA1, manufactured by Macrophi Inc., Lot 201107-7) is diluted by 800 times with PBS (-) that is filtration sterilized at the time of use. Each 50 µL is dispensed into a 96-well immunoplate, sealing with parafilm is provided to prevent evaporation of liquid, and preservation at 4°C is performed.
2) The 96-well immunoplate to which 34-G2 was added is taken out of the preservation at 4°C and after discarding the liquid, a blocking solution (a solution with which BSA is added to PBS (-) to achieve a final concentration of 3% (w/v)) is added at a proportion of 200 µL/well and leaving to stand at 25°C is performed for not less than 30 minutes to prevent nonspecific adsorption of antibodies and other proteins.
3) After washing three times with 200 µL of a washing solution (product equivalent to 10 mM Tris-HCl pH 7.5, 150 mM NaCl, and 0.05% (v/v) Tween 20), 50 µL portions of serially diluted LPS standard solutions (LPS standard substance (for example, *Pantoea agglomerans* derived LPS standard manufactured by Macrophi Inc., purity: not less than 95%)) are added to wells. 50 µL of a sample solution to be measured are added to another well of the same 96-well immunoplate. Leaving to stand in a thermostatic chamber at 25°C is performed for 1 hour.
4) The wells are washed three times with 200 µL of the washing solution, 4E-11 (primary antibody: antibody against O antigen polysaccharide of IP-PA1, manufactured by Macrophi Inc., Lot 20170702) diluted by 1,000 times is added at 50 µL/well, and leaving to stand in a thermostatic chamber at 25°C is performed for 1 hour.
5) The wells are washed three times with 200 µL of the washing solution, an alkaline phosphatase-conjugated anti mouse IgG immunoglobulin antibody (manufactured by Sigma, Cat. 1418) diluted by 1,000 times is added at 50 µL/well, and leaving to stand in a thermostatic chamber at 25°C is performed for 1 hour.
6) The wells are washed five times with 200 µL of the washing solution, a developing solution (disodium p-nitrophenyl phosphate hexahydrate (p-Npp); manufactured by Nacalai Tesque, Inc., Cat. 25019-52, Lot MOP2259) is added at 100 µL/well, and leaving to stand at room temperature is performed for 1 hour.
7) Thereafter, absorbances at 415 nm are measured with a microplate reader (Molecular Devices SpectraMax, Plus384). A calibration curve is prepared as a quadratic curve.
8) Using the calibration curve, an LPS concentration in the sample solution (µg/mL) is calculated from the absorbance of the sample solution. The LPS content (E) (µg/mg) in the sample is determined by dividing the LPS concentration in the sample solution by a sample concentration in the sample solution (mg/mL) (calculated from a sample weight value and a solution amount at the time of preparation).

Here, a detection limit of the ELISAmethod in the present measurement method is 1.6 ng/mL.

### (Method for quantification of LPS by Limulus test)

A quantity of an LPS by the Limulus test is determined by measuring an LPS content (L) (µg/mg) in a sample using, for example, a Limulus measurement kit that is capable of LPS-specific measurement. Specifically, the Limulus test can be performed by the following procedure.

### (LPS quantification method by Limulus test (endpoint chromogenic method))

Using a Limulus measurement kit that is capable of LPS-specific measurement (for example, Endospecy ES-50M Set, manufactured by SEIKAGAKU CORPORATION) in accordance with the attached instruction manual, the LPS content (L) (µg/mg) in a sample LPS to be measured is measured by the endpoint chromogenic method using a microplate.

For the measurement, CSE (10 ng/vial) (manufactured by SEIKAGAKU CORPORATION) is used as an endotoxin standard.

Based on the LPS contents measured by the two methods (E and L), the ratio thereof (E/L) can be determined. Here, a relationship between the structure and the molecular weight of an LPS shall first be described. As a structure, an LPS is constituted of O antigen polysaccharide, core polysaccharide, and lipid A portions. Among these, the molecular weight of the lipid A portion in one molecule of LPS does not differ by much among LPS types. The molecular weight of the core polysaccharide portion in one molecule of LPS also does not differ by much among LPS types. On the other hand, the O antigen polysaccharide portion in the LPS molecule is a repeated structure and the degree of repetition differs greatly among LPS types. As a result, differences in the molecular weight of the O antigen polysaccharide portion arise and differences in the molecular weight of LPS arise in accordance with the type of LPS. If the number of repetitions of the O antigen polysaccharide portion is high, the molecular weight of the LPS is high, and if the number of repetitions of the O antigen polysaccharide portion is low, the molecular weight of the LPS is low. Also, an LPS that is extracted from gram-negative bacterium is ordinarily a mixture of LPS of different molecular weights. Next, a relationship between the structure of the LPS and the LPS contents (E and L) shall be described. The LPS amount (E) that is measured by the ELISA method is an LPS amount that is determined based on the amount of antibodies that recognized and became bound to the O antigen polysaccharide in the LPS structure. If the number of repetitions of the O antigen polysaccharide in the LPS structure is low or there are no O antigen polysaccharides (that is, if the molecular weight of the LPS is low), antibody binding sites of sufficient size will not be secured within the LPS molecule, antibodies will not be able to bind to the LPS, and the measured value of the LPS amount by the ELISA method will be of small value. On the other hand, the LPS amount (L) measured by the Limulus test is an LPS amount that is determined based on reaction activity of the lipid A structure in the LPS structure and therefore, a measured value corresponding to the number of moles of LPS in the measured sample is determined regardless of the proportion or presence/non-presence of the O antigen polysaccharide in the LPS structure. Therefore, if when LPS contents (E and L) are determined by measuring an LPS sample (mixture of LPS differing in molecular weight) of fixed mass by the ELISA method and the endotoxin method, the E/L ratio is large, this means that the LPS contained in the measured sample contain a large amount of that of high molecular weight, and if the E/L ratio is small, this means that the LPS contained in the measured sample contain a large amount of that of low molecular weight.

The LPS obtained by the production method of the present invention is preferably such that the (E/L ratio) is more than 0 but not more than 1.0. Such an LPS is consequently high in content ratio of low molecular weight LPS in comparison to an LPS (IP-PA1) of the conventional production method. It is considered that in the production method of the present invention, there is a possibility that an LPS of high lipid solubility (an LPS having few hydrophilic carbohydrate chains and being high in proportion of the lipid-soluble lipid A portion) is selectively recovered because reverse-phase chromatography is used in the purification of the second step.

That is, by including the purification step that uses reverse-phase chromatography, an LPS of high content of low molecular weight LPS can be produced. An LPS that is high in safety and physiological activity (cytokine inducibility) due to being high in the content of low molecular weight LPS is thus obtained.

### Examples

Although the contents of the present invention shall now be described more specifically by way of examples and comparative examples, the scope of rights of the present invention is not restricted to the examples.

### <Preparation of gram-negative bacterium (Pantoea bacteria) used in the examples and comparative examples>

### (Preculture)

100 mL of a preculture medium solution (LB medium: 1% tryptone (special grade reagent for microorganism culturing, manufactured by Nacalai Tesque, Inc.), 0.5% yeast extract (for microorganism culturing, manufactured by Nacalai Tesque, Inc.), and 1% sodium chloride (special grade, manufactured by FUJIFILM Wako Pure Chemical Corporation)) were dispensed in each of (ten) 250 mL baffled flasks and sterilization at 121°C was performed for 15 minutes with an autoclave (HV-50, manufactured by HIRAYAMA). Inside a clean bench, *Pantoea agglomerans* (made by Macrophi Inc.) was sterilely inoculated from an agar medium into the flasks and shaking culturing was performed by setting in a shaking incubator (RMS-20R, manufactured by Sanki Seiki Co., Ltd.). The shaking culturing conditions were set to 35°C and 150 rpm and culturing was performed for 22 hours.

### (Main culture)

10 L of a main culture medium (1% tryptone, 0.5 % yeast extract, 1% sodium chloride, 0.2% glycerin (manufactured by FUJIFILM Wako Pure Chemical Corporation), 0.04% citric acid monohydrate (special grade, manufactured by FUJIFILM Wako Pure Chemical Corporation), 0.2% dipotassium hydrogen phosphate (special grade, manufactured by FUJIFILM Wako Pure Chemical Corporation), 0.07% ammonium hydrogen phosphate tetrahydrate (primary grade, manufactured by FUJIFILM Wako Pure Chemical Corporation), and 0.01% Antifoam 204 (manufactured by Sigma)) were placed in a 20 L jar fermenter (manufactured by B. E. Marubishi Co., Ltd.) and autoclave sterilization at 121°C was performed for 30 minutes.

To the main culture medium described above, 10% magnesium sulfate heptahydrate (special grade, manufactured by FUJIFILM Wako Pure Chemical Corporation) was added sterilely such as to be 0.04%. Further, 500 mL of the preculture medium described above were poured in sterilely to inoculate and main culturing was performed. The main culturing conditions were set to culturing temperature: 30°C; stirring speed: 300 rpm; aeration rate: 18 to 20 L/min; and pH: 6.8 to 7.2. The culture medium solution was recovered after 16 hours of culturing.

The main culturing operation of the 10 L medium scale described above was performed in parallel on two sets that were thereafter put together, homogenized (mixed), and then subject to following bacterial cell recovery operation.

### (Bacterial cell recovery)

The homogenized main culture medium solution described above was transferred into (20) 1 L centrifuge tubes and after performing centrifugation (high speed refrigerated centrifuge; 7780 manufactured by KUBOTA Corporation) under conditions of 4°C, 8,000 rpm, and 20 minutes, the supernatant was removed and a bacterial cell pellet was recovered as precipitate (total amount of the bacterial cell pellet: 260 g). The bacterial cell pellet obtained was cryopreserved at -30°C.

The bacterial cell pellet (cryopreserved product) of *Pantoea agglomerans* obtained by the above method was subject to extraction and purification of LPS by the methods of the examples or comparative examples.

### (Example 1) (Hot water extraction (first step) + ultrafiltration + C4 reverse-phase HPLC (second step))

### [1] Hot water extraction

78 g of the bacterial cell pellet of *Pantoea agglomerans* obtained by the operation of preparation of gram-negative bacterium (*Pantoea* bacteria) described above were added to and suspended in 400 mL of water for injection (manufactured by Otsuka Pharmaceutical Factory, Inc.). The pH of the suspension was measured by a pH meter (LAQUA, manufactured by Horiba, Ltd.) to be 6.0 and therefore, sodium hydroxide (manufactured by FUJIFILM Wako Pure Chemical Corporation) was added to neutralize. Thereafter, heating at 90°C was performed for 20 minutes using an autoclave (HV-50, manufactured by HIRAYAMA) . After cooling, the suspension was transferred to a 1 L centrifuge tube, centrifugation at 8,000 rpm was performed for 20 minutes, and the supernatant (hot water extract liquid) was recovered.

### [2] Ultrafiltration treatment

15 mL of the above hot water extract liquid were added to an ultrafiltration tube with a molecular weight cutoff of 30 kda (Amicon Ultra centrifugal filter units) and centrifuged (7,000 rpm × 30 minutes) to obtain a concentrated liquid containing LPS at the non-permeate side of the ultrafiltration membrane. Further, 15 mL of water for injection were added to the concentrated liquid and centrifugation was performed in the same manner to wash the concentrated liquid. Washing in the same manner was further repeated five times and then the washed concentrated liquid was recovered and diluted in a volumetric flask by adding water for injection to obtain 15 mL of an ultrafiltered suspension.

### [3] Pretreatment (solubilization treatment) for chromatography purification

1 mL of the above ultrafiltered suspension was diluted by 4 times by adding 3 mL of a solubilization buffer (0.25% sodium deoxycholate, 200 mM sodium chloride, and 10 mM tris-HCL (pH: approximately 9.5)) and mixing well. Non-solubilized precipitate was removed by a syringe filter of 0.45 µm pore diameter to obtain approximately 3.5mL of solubilized solution (solution containing the LPS in the abovementioned extract liquid) .

### [4] Chromatography purification

A portion (1500 µL) of the above solubilized solution was injected into a high performance liquid chromatography (HPLC) device and a component corresponding to LPS (sample fraction) was isolated. As the column, a reverse-phase silica gel column having a butyl group (alkyl group of 4 carbons) (C4 reverse-phase column) was used. That is, HPLC provided with a C4 reverse-phase column (C4 reverse-phase HPLC) was used. In regard to the mobile phase, an aqueous solution/methanol based gradient separation that is low in environmental burden was adopted. Formic acid and triethylamine were added to the aqueous solution. In regard to the gradient, gradient conditions were set such that after performing flow under a liquid A rich condition, a concentration of a liquid B is increased.

The specific separation conditions are as follows.

### <HPLC separation conditions>

· Device: HPLC ("Prominence," manufactured by Shimadzu Corporation)
· C4 reverse-phase column: XBridge Protein BEH C4 Column, 300 Å, 3.5 µm, 4.6 mm × 100 mm, manufactured by Waters
· Eluent: Liquid A aqueous solution of (50 mM formic acid/50 mM triethylamine)
   Liquid B methanol
· Gradient conditions (volume %):
   0 to 10 min: Fixed concentration conditions (Liquid A 95%/Liquid B 5%),
   10 to 35 min: Linear gradient conditions (Liquid A 95%/Liquid B 5% (10 min) → (Liquid A 5%/Liquid B 95% (35 min)),
   35 to 50 min: Fixed concentration (Liquid A 5%/Liquid B 95%)
· Flow rate: 4.5 mL/min
· Column temperature: 40°C
· Sample injection amount: 1500 µL
· Detector (DAD): 210 nm

### [5] Posttreatment (organic solvent removal and concentration) of chromatography purification

With the sample fraction isolated by the chromatography purification of [4] above, evaporation at 35°C (rotary evaporator) was performed for methanol removal and the aqueous solution that did not volatilize was recovered and diluted to 15 mL by adding water for injection.

This liquid was added to an ultrafiltration tube with a molecular weight cutoff of 10 kda (Amicon Ultra centrifugal filter units) and centrifuged (7,000 rpm × 30 minutes) to obtain a concentrated liquid containing LPS at the non-permeate side of the ultrafiltration membrane. Further, 15 mL of water for injection were added to the concentrated liquid and centrifugation was performed in the same manner to wash the concentrated liquid. Washing in the same manner was further repeated five times and then the washed concentrated liquid was recovered and diluted in a volumetric flask by adding water for injection to obtain 4 mL of an aqueous solution.

### [6] Freeze drying

The aqueous solution obtained was freeze dried with a vacuum freeze dryer (FD-2BU-SQ, manufactured by Nihon Techno Service Co., Ltd.) (conditions: -20°C, 5 Pa, 15 h) to obtain an LPS according to Example 1 as a final product.

### (Example 2) (Hot water extraction + C4 reverse-phase HPLC)

The hot water extract liquid obtained through step [1] of Example 1 was used. Step [2] ultrafiltration treatment that was performed with Example 1 was not performed.

Besides using 1 mL of the suspension after hot water extraction in place of the 1 mL of ultrafiltered suspension used in step [3] pretreatment (solubilization treatment) for chromatography purification of Example 1, the same operation as that of step [3] of Example 1 was performed to obtain approximately 3.5 mL of a solubilized solution.

The same operations as those of steps [4] chromatography purification, [5] posttreatment (organic solvent removal and concentration) of chromatography purification, and [6] freeze drying of Example 1 were performed on the solubilized solution obtained to obtain an LPS according to Example 2 as a final product.

### (Comparative Example 1) (TCA extraction + PhOH extraction + ultrafiltration)

### [11] TCA extraction

130 g of the bacterial cell pellet of *Pantoea agglomerans* obtained by the operation of preparation of gram-negative bacterium (*Pantoea* bacteria) described above and distilled water were added to a 3 L beaker and mixed by stirring to prepare a 300 mg/mL suspension (433 mL).

0.5 M trichloroacetic acid (TCA) aqueous solution of equal amount as the suspension was added and mixed by stirring at 15 to 20°C for 3 hours (100 rpm). Thereafter, transfer into a centrifuge tube and centrifugation (8,000 G × 15 minutes) were performed and a supernatant was obtained. This was added to a 3 L beaker and 10 N NaOH (20 µL/mL) was added to adjust the pH to 7.0 and a TCA extract liquid was thus obtained.

600 mL of ethanol at -20°C were added to 300 mL of the TCA extract liquid obtained and after mixing by stirring, the TCA extract liquid was left to stand overnight at -20°C, centrifugation (7,000 G × 15 minutes) was performed on the next day, and a precipitate was obtained. 40 mL of distilled water were added to the precipitate obtained to prepare a suspension, freeze drying was performed, and 1.7 g of dry TCA extract was obtained.

### [12] Phenol extraction

1.6 g of the dry TCA extract obtained and 40 mL of distilled water were added to a beaker and mixed by stirring to prepare a 40 mg/mL suspension. Next, 40 mL of a 90% phenol aqueous solution heated to 68°C were added to the suspension and stirring at 68°C was performed for 20 minutes. Next, after the suspension cooled to 20°C or less, the suspension was transferred into a centrifuge tube and centrifuged at room temperature (7,000 G × 30 minutes). An upper layer (1st extract liquid) in the centrifuge tube after centrifugation was recovered and a lower layer was returned to the beaker used for phenol extraction.

40 mL of distilled water were added to the abovementioned beaker and stirring at 68°C was performed for 20 minutes. Next, after the suspension cooled to 20°C or less, the suspension obtained was transferred into a centrifuge tube and centrifuged at room temperature (7,000 G × 30 minutes). An upper layer (2nd extract liquid) in the centrifuge tube after centrifugation was recovered, put together with the 1st extract liquid, and this was used as a phenol extract aqueous layer liquid.

### [13] Ultrafiltration treatment

The entire amount (approximately 80 mL) of the phenol extract aqueous layer liquid of [12] above was added to an ultrafiltration tube with a molecular weight cutoff of 10 kda (Amicon Ultra centrifugal filter units) and centrifuged (7,000 rpm × 30 minutes) to obtain a concentrated liquid containing LPS at the non-permeate side of the ultrafiltration membrane. Further, 15 mL of water for injection were added to the concentrated liquid and centrifugation was performed in the same manner to wash the concentrated liquid. Washing in the same manner was further repeated 11 times and then the washed concentrated liquid was recovered and diluted in a volumetric flask by adding water for injection to obtain 15 mL of an ultrafiltered suspension.

### Freeze drying

The ultrafiltered suspension obtained was freeze dried with a vacuum freeze dryer (FD-2BU-SQ, manufactured by Nihon Techno Service Co., Ltd.) (conditions: -20°C, 5 Pa, 15 h) to obtain an LPS according to Comparative Example 1 as a final product.

### (Comparative Example 2) (hot water extraction + ultrafiltration)

Using 15 mL of the hot water extract liquid obtained through step [1] of Example 1, the same operation as step [2] of Example 1 was performed to perform ultrafiltration. 15 mL of suspension was obtained by the ultrafiltration.

The same operation as step [6] freeze drying of Example 1 was performed on the ultrafiltered suspension obtained to obtain an LPS according to Comparative Example 2 as a final product.

### (Comparative Example 3) (hot water extraction only)

The same operation as step [6] freeze drying of Example 1 was performed on the hot water extract liquid obtained through step [1] of Example 1 to obtain an LPS according to Comparative Example 3 as a final product.

### <Quantification of LPS>

The LPS (hereinafter referred to as "sample") according to each of the Examples and Comparative Examples obtained by the steps described above were dissolved in the following eluent and quantification of the LPS by an following HPLC method was performed. Also, LPS yields with respect to 1 kg of bacterial cell pellet were calculated from the quantification results and these are indicated in Table 1 below.

### <HPLC method>

Each sample was diluted as suited with the eluent and injected into an HPLC. Quantification was performed by an absolute calibration curve method.

### <HPLC separation conditions>

· Device: HPLC ("HPLC 1260 Infinity," manufactured by Agilent Technologies, Inc.)
· Column: PL-aquagel-OH 30, manufactured by Agilent Technologies, Inc. (particle diameter: 8 µm; length: 300 mm × inner diameter: 7.5 mm) × 3 columns connected
· Eluent: 0.25% sodium deoxycholate, 200 mM sodium chloride, 10 mM tris-HCl aqueous solution (pH 9.5)
· Flow rate: 1.0 mL/min
· Column temperature: 40°C
· Sample injection amount: 50 µL
· Detector: Differential refractive index detector
· Standard substance: *Pantoea agglomerans* derived LPS standard (purity: not less than 95%)

### <Measurement of concentrations of proteins and nucleic acids in each sample>

Concentrations of proteins and nucleic acids remaining in each sample were measured by the following methods. The results are indicated in Table 1 below.

### (Measurement of protein content percentage)

Each weighed sample was dissolved in water and a protein content was measured by a BCA method ("Pierce BCA Protein Assay Kit," manufactured by Thermo).

Further, the protein content was determined by multiplying by a liquid volume of the aqueous solution.

Thereupon, a protein content percentage in each sample was determined by the following Math. 1 and is shown in Table 1. Protein content percentage (%) = (Protein content (mg) / Sample (mg)) × 100

### (Measurement of nucleic acid content percentage)

For aqueous solutions of respective weighed samples dissolved in water, absorbances at 260 nm and 320 nm of each sample aqueous solution were determined with a spectrophotometer (SpectraMax Plus 384, manufactured by Molecular Devices LLC.) using water as a reference and a nucleic acid concentration Ac in the aqueous solution was determined by the following Math. 2. Ac (µg/mL) = O. D. 260 nm - O. D. 320 nm) × 50

Further, multiplication by a liquid volume of the aqueous solution was performed to determine a nucleic acid content.

Thereupon, a nucleic acid content percentage in each sample was determined by the following Math. 3 and is shown in Table 1. Nucleic acid content percentage (%) = (Nucleic acid content (mg) / Sample (mg)) × 100

### (Calculation of LPS purity)

A purity of the LPS of each sample obtained was determined by Math. 4 and is shown in Table 1. LPS purity (%) = 100 - protein content percentage (%) - nucleic acid content percentage (%)

### (Method for calculating LPS yield per 1 kg of bacterial cell pellet)

If WLPS (g) of LPS was obtained by purifying v (mL) out of V (mL) of extract liquid (hot water or TCA) obtained from W (g) of bacterial cell pellet, an LPS yield per 1 kg of bacterial cell pellet is WLPS × (V/v) × (1,000/W) (g). Based on this, yields of the LPS according to the Examples and Comparative Examples were calculated and are shown in Table 1.

### <Class I designated chemical substance maximum treatment concentration>

### (Method for calculating class I designated chemical substance maximum treatment concentration)

If in each step, a (mL) of a solvent component A, b (mL) of a component B, and c (mL) of component C are mixed and B is a class I designated chemical substance, a class I designated chemical substance treatment concentration is {b/(a+b+c)} × 100 (%). The class I designated chemical substance treatment concentrations in the respective steps adopted in the Examples and Comparative Examples were calculated and those of the highest value are shown in Table 1 as class I designated chemical substance maximum treatment concentrations.

Here, the substances used in preparing the sample according to Comparative Example 1 that are designated as class I designated chemical substances (Act on Confirmation, etc. of Release Amounts of Specific Chemical Substances in the Environment and Promotion of Improvements to the Management Thereof) are trichloroacetic acid and phenol and the maximum treatment concentrations of these in the production process are shown in Table 1 below.

Also, the substance used in preparing the samples according to Example 1 and Example 2 that is designated as a class I designated chemical substance (Act on Confirmation, etc. of Release Amounts of Specific Chemical Substances in the Environment and Promotion of Improvements to the Management Thereof) is triethylamine and the maximum treatment concentrations in the production processes are shown in Table 1 below.

| | Process | Extraction solvent | Class I designated chemical substance maximum treatment concentration (w/w%) | LPS yield g / 1 kg bacterial cell pellet | LPS purity (w/w%) | Protein content percentage | Nucleic acid content percentage |
|---|---|---|---|---|---|---|---|
| | | | | | | (w/w%) | (w/w%) |
| Example 1 | Hot water extraction | Water | 0.5 | 6.4 | 94.7 | 5 | 0.3 |
| | + Ultrafiltration | | | | | | |
| | + C4 reverse phase HPLC | | | | | | |
| Example 2 | Hot water extraction | Water | 0.5 | 6.9 | 94.4 | 5.2 | 0.4 |
| | + C4 reverse phase HPLC | | | | | | |
| Comparative Example 1 | TCA extraction | TCA + PhOH + Water | 45 | 10 | 97.6 | 0.51 | 1.86 |
| | + PhOH extraction | | | | | | |
| | + Ultrafiltration | | | | | | |
| Comparative Example 2 | Hot water extraction | Water | 0 | 3.2 | 49.7 | 23.1 | 27.2 |
| | + Ultrafiltration | | | | | | |
| Comparative Example 3 | Hot water extraction only | Water | 0 | 0.9 | 20.1 | 63.5 | 16.4 |

According to the results of Table 1, in Examples 1 and 2, it was made possible by a combination of hot water extraction and C4 reverse-phase column HPLC to produce LPS of sufficient purity regardless of performing or not performing ultrafiltration, without using an organic solvent of high environmental burden as the extraction solvent, and also while keeping low a usage amount of a class I designated chemical substance in all steps of the production method. It can be understood that high purity that is approximately the same as and also a yield comparable to Comparative Example 1, which is a conventional LPS production method with extraction being performed with TCA and phenol that are high in environmental burden, are achieved. It can also be understood that with hot water extraction only or with just hot water extraction and ultrafiltration, the LPS yield and the LPS purity are both insufficient. From these results, it is clear that by combining hot water extraction and HPLC using a specific reverse-phase column, it is possible to produce an LPS at satisfactory levels of both LPS yield and LPS purity without using an organic solvent of high environmental burden in the extraction step and also while keeping low the usage amount of a class I designated chemical substance in the production method as a whole.

Next, in regard to the LPS (LPS purity: 94.4%) prepared by the production method of Example 2, a molecular weight distribution was evaluated by an SDS-PAGE gel electrophoresis method for the purpose of clarifying structural features as LPS. Also, an LPS quantitative value (E) determined by an ELISA method and an LPS quantitative value (L) determined by a Limulus test (endpoint chromogenic method) were determined and a ratio thereof (E/L) was evaluated.

The measurement methods and the measurement results are described below.

### (Measured samples)

Sample prepared by the production method of Example 2 (LPS purity: 94.4%) (Lot 2 and Lot 3)

IP-PA1 standard (*Pantoea agglomerans* derived LPS, LPS purity: 97.3%, manufactured by Macrophi Inc., Lot 4) (measured for comparison)

Here, although the IP-PA1 standard is a *Pantoea agglomerans* derived LPS, its production method differs from the present invention. IP-PA1 is obtained by extraction by hot phenol water and purification by anion exchange chromatography.

### <Measurement of molecular weight distribution by SDS-PAGE gel electrophoresis method>

### (Measurement method)

The LPS was fractionated by Tricine-SDS-PAGE and made visible by a silver staining method. For the Tricine-SDS-PAGE, the paper (1) by Schaegger et al. was referenced. As a 15% polyacrylamide gel, a precast gel manufactured by ATTO Corporation was used, and tricine was added just to an electrophoresis buffer. For the silver staining method, the paper (2) by Tsai et al. was referenced and staining was performed using the Electrophoresis Silver Staining II Kit Wako manufactured by FUJIFILM Wako Pure Chemical Corporation.

References
(1) Tricine-sodium dodecyl sulfate-polyacrylamide gel electrophoresis for the separation of proteins in the range from 1 to 100 kDa. Schaegger H, von Jagow G. Anal Biochem. 1987 Nov 1; 166(2): 368-79
(2) A sensitive silver stain for detecting lipopolysaccharides in polyacrylamide gels. Tsai CM, Frasch CE. Anal Biochem. 1982 Jan 1; 119(1): 115-9.

### (Preparation and electrophoresis of electrophoresis sample solutions)

The IP-PA1 standard and the LPS of Example 2 (Lot 2 and Lot 3) were electrophoresed.

The IP-PA1 standard and the LPS of Example 2 (Lot 2 and Lot 3) were each prepared to 500 µg/mL by adding distilled water. From each, three portions of 12 µL each were respectively dispensed into 1.5 mL tubes, added and mixed well with an equal amount (12 µL) of a sample buffer (sample buffer (for SDS-PAGE, 2 times concentrated, contains 2-ME), manufactured by Nacalai Tesque, Inc.), and heat treated for 5 minutes inside a heating block (aluminum block heater, manufactured by SCINICS Corporation) at 100°C. After treatment, rapid cooling in ice water was performed and after collecting each sample at the bottom of the tube by centrifugation, 20 µL were applied to the gel.

The electrophoresis buffer was prepared to the composition of Table 2 below.

**[Table 2]**

| Electrophoresis buffer (pH 8.4) | |
|---|---|
| Tricine | 8.95 g |
| Tris | 1.975 g |
| 10% SDS aqueous solution | 5 mL |
| Dw | up to 500 mL |

With each size marker (Precision Plus Protein Dual Xtra Standards, molecular weight sizes: 250, 150, 100, 75, 50, 37, 25, 20, 15, 10, 5, and 2 kDa, manufactured by Bio-Rad Laboratories, Inc.), 1 µL was added to a sample buffer (sample buffer (for SDS-PAGE, 2 times concentrated, contains 2-ME), manufactured by Nacalai Tesque, Inc.) that was diluted by 2 times with distilled water (39 µL) and 20 µL of this was applied to the gel. Immediately after application of the size markers and the samples, electrophoresis at 20 mA constant current was started and the electrophoresis was ended at the point at which a BPB (marker dye) was electrophoresed to a position 1 cm from a lower end of the separation gel (approximately 60 minutes) . After the end of electrophoresis, the gel plate was removed from the device and the gel was immersed in a fixing solution for silver staining.

### (Silver staining method)

Staining was performed using the Electrophoresis Silver Staining II Kit Wako manufactured by FUJIFILM Wako Pure Chemical Corporation. The staining operation was performed in accordance with the attached instruction manual.

The electrophoretogram obtained is shown in the SDS-PAGE (silver staining method) electrophoretogram of FIG. 1. With the SDS-PAGE of FIG. 1, IP-PA1 standard (Lot 4) (N = 3), the LPS of Example 2 (Lot 2) (N = 3), the LPS of Example 2 (Lot 3) (N = 3), and the protein size markers are shown in that order from the left side. The ordinate of FIG. 1 indicates the molecular weight. The molecular weights determined on the basis of the protein markers are shown in Table 3 below.

### (Analysis of total luminance values of respective bands)

Next, using ImageJ Software (free image processing software; http://imagej.nih.gov/ij/; ImageJ 1.52a), image data were read and total luminance values (luminance value × area) of the high molecular weight area band and the low molecular weight area band of the respective lanes were determined. The measurement results of the total luminance values of the low molecular weight component and the high molecular weight component obtained by SDS-PAGE are shown in Table 4 below.

**[Table 3]**

| Sample | Lot | Low molecular weight | High molecular weight |
|---|---|---|---|
| | | Lower limit to upper limit | Lower limit to upper limit |
| IP-PA1 standard | Lot 4 | 5,000 to 20,000 | 20,000 to 70,000 |
| LPS of Example 2 | Lot 2 | 5,000 to 20,000 | 25,000 to 60,000 |
| | Lot 3 | 5,000 to 20,000 | 25,000 to 60,000 |

| Sample | Lot | Total luminance value of high molecular weight area | Total luminance value of low molecular weight area | Total | Proportion of total luminance value of low molecular weight area (%) | | |
|---|---|---|---|---|---|---|---|
| | | (H) | (L) | (H+L) | (100×L /(H+L)) | Average | SD |
| IP-PA1 standard | Lot 4 | 3,257,440 | 9,555,869 | 12,813,309 | 74.6 | 74.9 | 0.5 |
| | | 3,105,610 | 9,556,927 | 12,662,537 | 75.5 | | |
| | | 3,310,321 | 9,693,134 | 13,003,455 | 74.5 | | |
| LPS of Example 2 | Lot 2 | 1,776,427 | 8,429,848 | 10,206,275 | 82.6 | 82.3 | 0.3 |
| | | 1,861,574 | 8,440,484 | 10,302,058 | 81.9 | | |
| | | 1,800,328 | 8,407,151 | 10,207,479 | 82.4 | | |
| | Lot 3 | 1,423,950 | 7,844,492 | 9,268,442 | 84.6 | 84.6 | 0.3 |
| | | 1,382,482 | 7,732,432 | 9,114,914 | 84.8 | | |
| | | 1,419,056 | 7,560,887 | 8,979,943 | 84.2 | | |

### (Measurement results)

As results of electrophoresis, the molecular weight determined in the basis of the protein markers was 5,000 to 20,000 for the low molecular weight area bands of both the IP-PA1 standard and the LPS of Example 2. In regard to the high molecular weight area bands, whereas that of the IP-PA1 standard was of 20,000 to 70,000, that of the LPS of Example 2 was of a narrow distribution of 25,000 to 60,000.

Next, in regard to the total luminance values of the respective bands, whereas with the IP-PA1 standard, the proportion of the low molecular weight LPS according to luminance value was 74.9%, that of LPS Lot 2 of Example 2 was 82.3% and that of Lot 3 was 84.6%, thus showing that with both lots of the LPS of Example 2, the percentage of the low molecular weight LPS is higher than that of the IP-PA1 standard.

### <Measurement of E/L ratio>

### (Method for quantification of LPS by ELISA method)

The LPS content (E) (µg/mg) in each sample was measured by the ELISA method using an IP-PA1 carbohydrate specific antibody (manufactured by Macrophi Inc.). Specifically, the ELISA measurement was performed by the following procedure.
1) 34-G2 (immobilized antibody: antibody against O antigen polysaccharide of IP-PA1, manufactured by Macrophi Inc., Lot 201107-7) was diluted by 800 times with PBS (-) that was filtration sterilized at the time of use. Aliquots of 50 µL each were dispensed into a 96-well immunoplate, sealing with parafilm was provided to prevent evaporation of liquid, and preservation at 4°C was performed.
2) The 96-well immunoplate to which 34-G2 was added was taken out of the preservation at 4°C and after discarding the liquid, a blocking solution (a solution with which BSA is added to PBS (-) to achieve a final concentration of 3% (w/v)) was added at a proportion of 200 µL/well and leaving to stand at 25°C was performed for not less than 30 minutes to prevent nonspecific adsorption of antibodies and other proteins.
3) After washing three times with 200 µL of a washing solution (product equivalent to 10 mM Tris-HCl pH 7.5, 150 mM NaCl, and 0.05% (v/v) Tween 20), 50 µL portions of serially diluted IP-PA1 standard (IP-PA1, purity: not less than 97.2%, manufactured by Macrophi Inc., Lot 4) were added to wells. 50 µL of a sample solution to be measured were added to another well of the same 96-well immunoplate. Leaving to stand in a thermostatic chamber at 25°C was performed for 1 hour.
4) The wells were washed three times with 200 µL of the washing solution, 4E-11 (primary antibody: antibody against O antigen polysaccharide of IP-PA1, manufactured by Macrophi Inc., Lot 20170702) diluted by 1,000 times was added at 50 µL/well, and leaving to stand in a thermostatic chamber at 25°C was performed for 1 hour.
5) The wells were washed three times with 200 µL of the washing solution, an alkaline phosphatase-conjugated anti mouse IgG immunoglobulin antibody (manufactured by Sigma, Cat. 1418) diluted by 1,000 times was added at 50 µL/well, and leaving to stand in a thermostatic chamber at 25°C was performed for 1 hour.
6) The wells were washed five times with 200 µL of the washing solution, a developing solution (disodium p-nitrophenyl phosphate hexahydrate (p-Npp); manufactured by Nacalai Tesque, Inc., Cat. 25019-52, Lot MOP2259) was added at 100 µL/well, and leaving to stand at room temperature was performed for 1 hour.
7) Thereafter, absorbances at 415 nm were measured with a microplate reader (Molecular Devices SpectraMax, Plus384). A calibration curve was prepared as a quadratic curve.
8) Using the calibration curve, an LPS concentration in the sample solution (µg/mL) was calculated from the absorbance of the sample solution. The LPS content (E) (µg/mg) in the sample was determined by dividing the LPS concentration in the sample solution by a sample concentration in the sample solution (mg/mL) (calculated from a sample weight value and a solution amount at the time of preparation).

Here, a detection limit of the present ELISA method is 1.6 ng/mL.

The LPS quantification by the ELISA method was performed on the IP-PA1 standard (Lot 4) and the LPS of Example 2 (Lot 2 and Lot 3). The respective measurement results are shown in Table 5 below.

### (LPS quantification method by Limulus test (endpoint chromogenic method))

Using a Limulus measurement kit that is capable of LPS-specific measurement (Endospecy ES-50M Set, manufactured by SEIKAGAKU CORPORATION) in accordance with the attached instruction manual, the LPS contents (L) (µg/mg) in the samples (the LPS of Example 2 (Lot 2 and Lot 3) and the IP-PA1 standard (Lot 4)) were measured by the endpoint chromogenic method using a microplate.

For the measurement, CSE (10 ng/vial) (manufactured by SEIKAGAKU CORPORATION) is used as an endotoxin standard. The respective measurement results are shown in Table 5 below.

Based on the LPS contents measured by the two methods (E and L), the ratios thereof (E/L) were determined. The results are shown in Table 5 below.

**[Table 5]**

| Sample | Lot | Limulus (µg/mg) | ELISA (µg/mg) | E/L ratio |
|---|---|---|---|---|
| | | Mean ± SD | Mean ± SD | |
| IP-PA1 standard | Lot 4 | 608186 | 998±29 | 1.64 |
| LPS of Example 2 | Lot 2 | 1,016±45 | 727127 | 0.72 |
| | Lot 3 | 972±33 | 697±23 | 0.71 |

### (Measurement results)

Whereas the E/L ratio of IP-PA1 produced by the conventional production method is 1.64, the LPS of Example 2 exhibited low values of 0.71 to 0.72.

Since an LPS is an organism-derived substance, it is considered to be a mixture of molecules of different structures and molecular weights. The product of the present invention is thus difficult to define clearly by a structural formula. Characteristics of the produced product were thus clarified by evaluating characteristic values due to structure. That is, from the measurement results of the SDS-PAGE gel electrophoresis method, it was possible to confirm that by producing by the methods of the Examples, LPS mixtures were obtained with which the percentage of low molecular weight LPS with respect to the entirety of the LPS is higher than that of the LPS (IP-PA1) of the conventional production method. Also, that the value of the E/L ratios of the LPS produced by the methods of the Examples are lower than that of the LPS (IP-PA1) of the conventional production method indicates that a carbohydrate chain portion that is large in contribution to molecular weight is shortened and it was confirmed from this result as well that it is indicated that by producing by the methods of the Examples, LPS mixtures were obtained with which the percentage of low molecular weight LPS with respect to the entirety of the LPS is higher than that of the LPS (IP-PA1) of the conventional production method.

That is, it is considered that there is a possibility that an LPS of high lipid solubility (an LPS having few hydrophilic carbohydrate chains and being high in proportion of the lipid- soluble lipid A portion) is selectively recovered because reverse-phase chromatography is used in the purification step in the production method of the present invention.

As described above, with the LPS resulting from the production method of the present invention, a product (LPS) with a high proportion of low molecular weight LPS in the entirety of LPS is obtained by producing by a specific production method. And due to this molecular weight distribution, the LPS is that of high safety and physiological activity (cytokine inducibility).

## Claims

1. A lipopolysaccharide production method in which a lipopolysaccharide (LPS) is extracted and purified from gram-negative bacterium, the lipopolysaccharide production method comprising:
a first step of performing extraction from the gram-negative bacterium using hot water to obtain an extract liquid that contains the lipopolysaccharide, wherein a temperature of the hot water is 50 to 150°C; and
a second step of purifying the extract liquid or a solution containing the LPS in the extract liquid by using reverse-phase liquid chromatography to obtain the lipopolysaccharide,
wherein a reverse-phase column used in the reverse-phase liquid chromatography has a packing material constituted of a material having a functional group of 1 to 8 carbons.

2. The lipopolysaccharide production method according to Claim 1, wherein the packing material is constituted of a material having a functional group of 2 to 6 carbons.

3. The lipopolysaccharide production method according to Claim 1 or 2, wherein the packing material is constituted of a material having a functional group of 2 to 4 carbons.

4. The lipopolysaccharide production method according to any one of Claims 1 to 3, wherein the packing material is constituted of a material having a functional group of 4 carbons.

5. The lipopolysaccharide production method according to any one of Claims 1 to 4, wherein the functional group is an alkyl group.

6. The lipopolysaccharide production method according to any one of Claims 1 to 5, wherein a temperature of the hot water of the first step is 50 to 99°C.

7. The lipopolysaccharide production method according to any one of Claims 1 to 6, wherein a temperature of the hot water of the first step is 70 to 99°C.

8. The lipopolysaccharide production method according to any one of Claims 1 to 7, wherein a temperature of the hot water of the first step is 85 to 95°C.

9. The lipopolysaccharide production method according to any one of Claims 1 to 8, wherein the gram-negative bacterium is at least one selected from the group consisting of a genus *Escherichia, Salmonella, Pantoea, Acetobacter, Zymomonas, Xanthomonas, Enterobacter, Roseomonas,* and *Rhodobactor.*

10. The lipopolysaccharide production method according to any one of Claims 1 to 9, wherein the gram-negative bacterium belongs to the genus *Pantoea.*

## Patentansprüche

1. Verfahren zur Herstellung von Lipopolysacchariden, bei dem ein Lipopolysaccharid (LPS) aus einem gramnegativen Bakterium extrahiert und aufgereinigt wird, wobei das Verfahren zur Herstellung von Lipopolysacchariden umfasst:
einen ersten Schritt des Durchführens einer Extraktion aus dem gramnegativen Bakterium unter Verwendung von heißem Wasser, um eine Extraktflüssigkeit zu erhalten, die das Lipopolysaccharid enthält, wobei eine Temperatur des heißen Wassers 50 bis 150 °C beträgt; und
einen zweiten Schritt des Aufreinigens der Extraktflüssigkeit oder einer Lösung, die das LPS in der Extraktflüssigkeit enthält, unter Verwendung von Umkehrphasen-Flüssigchromatographie, um das Lipopolysaccharid zu erhalten,
wobei eine Umkehrphasen-Säule, die in der Umkehrphasen-Flüssigchromatographie verwendet wird, ein Packungsmaterial aufweist, das aus einem Material mit einer funktionellen Gruppe mit 1 bis 8 Kohlenstoffatomen besteht.

2. Verfahren zur Herstellung von Lipopolysacchariden nach Anspruch 1, wobei das Packungsmaterial aus einem Material mit einer funktionellen Gruppe mit 2 bis 6 Kohlenstoffatomen besteht.

3. Verfahren zur Herstellung von Lipopolysacchariden nach Anspruch 1 oder 2, wobei das Packungsmaterial aus einem Material mit einer funktionellen Gruppe mit 2 bis 4 Kohlenstoffatomen besteht.

4. Verfahren zur Herstellung von Lipopolysacchariden nach einem der Ansprüche 1 bis 3, wobei das Packungsmaterial aus einem Material mit einer funktionellen Gruppe mit 4 Kohlenstoffatomen besteht.

5. Verfahren zur Herstellung von Lipopolysacchariden nach einem der Ansprüche 1 bis 4, wobei die funktionelle Gruppe eine Alkylgruppe ist.

6. Verfahren zur Herstellung von Lipopolysacchariden nach einem der Ansprüche 1 bis 5, wobei eine Temperatur des heißen Wassers des ersten Schritts 50 bis 99°C beträgt.

7. Verfahren zur Herstellung von Lipopolysacchariden nach einem der Ansprüche 1 bis 6, wobei eine Temperatur des heißen Wassers des ersten Schritts 70 bis 99°C beträgt.

8. Verfahren zur Herstellung von Lipopolysacchariden nach einem der Ansprüche 1 bis 7, wobei eine Temperatur des heißen Wassers des ersten Schritts 85 bis 95°C beträgt.

9. Verfahren zur Herstellung von Lipopolysacchariden nach einem der Ansprüche 1 bis 8, wobei das gramnegative Bakterium mindestens eines ist, das aus der Gruppe ausgewählt ist, die aus den Gattungen *Escherichia, Salmonella, Pantoea, Acetobacter, Zymomonas, Xanthomonas, Enterobacter, Roseomonas* und *Rhodobactor* besteht.

10. Verfahren zur Herstellung von Lipopolysacchariden nach einem der Ansprüche 1 bis 9, wobei das gramnegative Bakterium zur Gattung *Pantoea* gehört.

## Revendications

1. Procédé de production de lipopolysaccharides dans lequel un lipopolysaccharide (LPS) est extrait et purifié à partir d'une bactérie à gram négatif, le procédé de production de lipopolysaccharides comprenant :
une première étape consistant à effectuer une extraction à partir de la bactérie à gram négatif en utilisant de l'eau chaude pour obtenir un extrait liquide qui contient le lipopolysaccharide, dans lequel une température de l'eau chaude est de 50 à 150 °C ; et
une seconde étape consistant à purifier l'extrait liquide ou une solution contenant le LPS dans l'extrait liquide en utilisant une chromatographie liquide en phase inverse pour obtenir le lipopolysaccharide,
dans lequel une colonne en phase inverse utilisée dans la chromatographie liquide en phase inverse présente un matériau de remplissage constitué d'un matériau présentant un groupe fonctionnel de 1 à 8 carbones.

2. Procédé de production de lipopolysaccharides selon la revendication 1, dans lequel le matériau de remplissage est constitué d'un matériau présentant un groupe fonctionnel de 2 à 6 carbones.

3. Procédé de production de lipopolysaccharides selon la revendication 1 ou 2, dans lequel le matériau de remplissage est constitué d'un matériau présentant un groupe fonctionnel de 2 à 4 carbones.

4. Procédé de production de lipopolysaccharides selon l'une quelconque des revendications 1 à 3, dans lequel le matériau de remplissage est constitué d'un matériau présentant un groupe fonctionnel de 4 carbones.

5. Procédé de production de lipopolysaccharides selon l'une quelconque des revendications 1 à 4, dans lequel le groupe fonctionnel est un groupe alkyle.

6. Procédé de production de lipopolysaccharides selon l'une quelconque des revendications 1 à 5, dans lequel une température de l'eau chaude de la première étape est de 50 à 99 °C.

7. Procédé de production de lipopolysaccharides selon l'une quelconque des revendications 1 à 6, dans lequel une température de l'eau chaude de la première étape est de 70 à 99 °C.

8. Procédé de production de lipopolysaccharides selon l'une quelconque des revendications 1 à 7, dans lequel une température de l'eau chaude de la première étape est de 85 à 95 °C.

9. Procédé de production de lipopolysaccharides selon l'une quelconque des revendications 1 à 8, dans lequel la bactérie à gram négatif est au moins une choisie dans le groupe constitué d'un genre *Escherichia, Salmonella, Pantoea, Acetobacter, Zymomonas, Xanthomonas, Enterobacter, Roseomonas* et *Rhodobactor.*

10. Procédé de production de lipopolysaccharides selon l'une quelconque des revendications 1 à 9, dans lequel la bactérie à gram négatif appartient au genre *Pantoea.*
